# EUROPEAN PATENT SPECIFICATION

(11) **EP 2 211 845 B1**
(45) Date of publication and mention of the grant of the patent: **22.02.2017**
(21) Application number: 08805247.7
(22) Date of filing: 13.10.2008
(51) Int. Cl.: A61K 31/567

(54) **GLUCOCORTICOID RECEPTOR ANTAGONISTS SUCH AS MIFEPRISTONE FOR TREATING CUSHING'S SYNDROME**
GLUCOCORTICOID-REZEPTOR-ANTAGONISTEN WIE MIFEPRISTON ZUR BEHANDLUNG DES CUSHING-SYNDROMS
ANTAGONISTES DU RECEPTEUR GLUCOCORTICOIDE COMME MIFEPRISTONE TRAITEMENT DU SYNDROME DE CUSHING

(30) Priority: 17.10.2007 US 960856 P
(43) Date of publication of application: 04.08.2010
(60) Divisional application: 12183545.8
(73) Proprietor: Laboratoire HRA Pharma, 75003 Paris (FR)
(72) Inventor: ULMANN, André, F-75005 Paris (FR); GAINER, Erin, F-75019 Paris (FR); VUILLET, François, F-75017 Paris (FR)
(74) Representative: Chajmowicz, Marion
(86) International application number: PCT/EP2008/063699
(87) International publication number: WO 2009/050136

(56) References cited:
- WO-A-2004/041215
- WO-A-2005/070893
- WO-A-2005/087769
- WO-A-2006/014394
- WO-A-2008/060391
- LELY VAN DER A-J ET AL: "RAPID REVERSAL OF ACUTE PSYCHOSIS IN THE CUSHING SYNDROME WITH THE CORTISOL-RECEPTOR ANTAGONIST MIFEPRISTONE (RU 486)" ANNALS OF INTERNAL MEDICINE, NEW YORK, NY; US, US, vol. 114, no. 2, 15 January 1991 (1991-01-15), page 143/144, XP002041041 ISSN: 0003-4819
- CHU J W ET AL: "Successful long-term treatment of refractory Cushing's disease with high-dose mifepristone (RU 486)" JOURNAL OF CLINICAL ENDOCRINOLOGY AND METABOLISM, ENDOCRINE SOCIETY, CHEVY CHASE, MD, vol. 86, no. 8, 1 August 2001 (2001-08-01), pages 3568-3573, XP002410091 ISSN: 0021-972X
- BENAGIANO GIUSEPPE ET AL: "Selective progesterone receptor modulators 3: use in oncology, endocrinology and psychiatry." EXPERT OPINION ON PHARMACOTHERAPY OCT 2008, vol. 9, no. 14, October 2008 (2008-10), pages 2487-2496, XP009110411 ISSN: 1744-7666
- R CZERWONKA ET AL: 'RU 486 administration in a child with Cushing's syndrome' THE LANCET vol. 5, no. 8552, 25 July 1987, GB, page 217, XP055194811 ISSN: 0140-6736
- SARTOR O ET AL: "Mifepristone: Treatment of Cushing's syndrome", CLINICAL OBSTETRICS AND GYNECOLOGY, HARPER AND ROW, HAGERSTOWN, MD, US, vol. 39, no. 2, 1 January 1996 (1996-01-01), pages 506-510, XP009184753, ISSN: 0009-9201

## Description

The invention relates to a method for treating Cushing's syndrome using glucocorticoid receptor antagonists.

### BACKGROUND OF THE INVENTION

Cushing's syndrome of endogenous origin is a hormonal disease with an estimated incidence of approximately 10 per 1 million persons (Meier and Biller, 1997). Cushing's syndrome is associated with an increased blood concentration of cortisol (hypercortisolism) or the presence in blood of glucocorticoid hormone excess over a long period of time. Cushing's syndrome is classified as either ACTH dependent or non ACTH dependent.

ACTH dependent Cushing's syndrome is characterised by a chronic ACTH hypersecretion which stimulates the growth of the adrenal glands and the hypersecretion of corticosteroids. The most common underlying cause of ACTH dependent Cushing's syndrome is excessive production of ACTH by pituitary adenomas known as Cushing's disease. Cushing's syndrome resulting from the production of ACTH in another location than the pituitary gland is known as ectopic Cushing's syndrome. Examples of ectopic sites include thymoma, medullary carcinoma of the thyroid, pheochromocytoma, islet cell tumours of the pancreas and small cell carcinoma of the lung.

ACTH independent Cushing's syndromes are caused by adrenal tumors that can be either adenomas or carcinomas. Both adrenal adenomas and carcinomas are characterised by chronic cortisol hypersecretion.

Symptoms of Cushing's syndrome include a characteristic abnormal fat deposition around the neck, thinning of the skin, osteoporosis, moon face, weakness, fatigue, backache, headache, impotence, muscle atrophy, increased thirst, urination, insulin resistance, dyslipidemia, myopathy, amenorrhea, hypertension, weight gain, central obesity, steroid hypersecretion, elevated urinary cortisol excretion and mental status changes, in particular depression (Orth 1995; Dahia and Grossman, 1999).

Effective drug therapies for Cushing's syndrome currently are not satisfactory. The oral inhibitors of adrenal steroidogenesis are the most commonly used medical agents in the treatment of Cushing's syndrome: these include metyrapone, ketoconazole, aminoglutethimide, mitotane and trilostane.

In ectopic ACTH secretion, when the tumor cannot be found or removed, medical therapy may be used to reduce cortisol production (Doppman et al, 1987, Doppman et al, 1989, Pass et al , 1990, Wajchenberg et al, 1994, Newell-Price et al, 1998). Furthermore, clinical trials showed some efficacy using high-dose mifepristone once a day (Nieman et al, 1985; Chrousos et al, 1989; van der Lely, 1991, Newfield et al, 2000; Chu et al, 2001). A fractioned dosage of mifepristone was successfully given to a young child (Beaufrère et al, 1987).

However in a long term, such high dosage of mifepristone given with long intervals between doses (e.g. once a day) triggers a massive secretion of cortisol due to interruption of the endogenous feedback mechanism. This high level of cortisol then overwhelms the blockage of the glucocorticoid receptors, leading to hypercortisolism (Raux-Demay et al, 1990).

### SUMMARY OF THE INVENTION

In order to avoid secretion of cortisol in response to the blockade of the glucocorticoid receptor, it is now proposed to give multiple low doses of a glucocorticoid receptor antagonist, for treating Cushing's syndrome.

The invention more particularly provides a pharmaceutical composition comprising a mifepristone, for use in treating Cushing's syndrome in an adult or an adolescent patient, wherein said pharmaceutical composition is suitable for oral administration, and is administered at least three times a day, wherein the total daily amount of mifepristone administered is inferior or equal to 600mg, preferably wherein the total daily amount of mifepristone administered is less than about 20mg/kg/day.

### DETAILED DESCRIPTION OF THE INVENTION

### Definitions:

Unless otherwise indicated, the patient to be treated may be any human subject afflicted with Cushing's syndrome, whatever the sex and the age of the subject. The patient may be a child, an adolescent (i.e. generally a subject who is 12 years old or above), or an adult. The patient to be treated is afflicted with Cushing's syndrome, preferably caused by ectopic ACTH secretion.

### Multiple doses

It is herein described a method for treating Cushing's syndrome in an adult or an adolescent patient, which method comprises administering the patient with a pharmaceutical composition comprising mifepristone at least three times a day, e.g. three or four times a day.

Such chronic daily administration of the glucocorticoid receptor antagonist in subjects with Cushing's syndrome makes it possible to normalize glucocorticoid-dependent parameters through its cortisol-blocking action.

Preferably the daily dosage is less than about 40mg/kg/day, preferably less than about 20mg/kg/day.

The total daily amount of the glucocorticoid receptor antagonist administered may be advantageously inferior or equal to 600mg, still preferably inferior or equal to 400mg, still more preferably inferior or equal to 300mg.

The pharmaceutical composition is suitable for oral administration.

### Routes of administration:

For a brief review of present methods for drug delivery, see, Langer, Science 249:1527-1533 (1990). Methods for preparing administrable compounds are known or are apparent to those skilled in the art and are described in more detail in, for example, Remington's Pharmaceutical Science, 17th ed., Mack Publishing Company, Easton, Pa. (1985), which is hereinafter referred to as "Remington."

For solid compositions, conventional non toxic solid carriers may be used which include, for example, pharmaceutical grades of mannitol, lactose, starch, magnesium stearate, sodium saccharine, talcum, cellulose, glucose, sucrose, magnesium, carbonate, and the like. For oral administration, a pharmaceutically acceptable nontoxic composition is formed by incorporating any of the normally employed excipients, such as those carriers previously listed.

Oral solid dosage forms are preferentially compressed tablets or capsules. Compressed tablets may contain any of the excipients described above which are diluents to increase the bulk of the active ingredient so that production of a compressed tablet of practical size is possible. Binders, which are agents which impart cohesive qualities to powdered materials are also necessary. Starch, gelatine, sugars such as lactose or dextrose, and natural and synthetic gums are used. Disintegrants are necessary in the tablets to facilitate break-up of the tablet. Disintegrants include starches, clays, celluloses, algins, gums and crosslinked polymers. Lastly small amounts of materials known as lubricants and glidants are included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. Procedures for the production and manufacture of compressed tablets are well known by those skilled in the art (See Remington).

Capsules are solid dosage forms using preferentially either a hard or soft gelatine shell as a container for the mixture of the active ingredient and inert ingredients. Procedures for production and manufacture of hard gelatin and soft elastic capsules are well known in the art (See Remington).

Buccal forms or devices are also useful, such as those described in U.S. patent application 20050208129. U.S. patent application 20050208129 describes a prolonged release bioadhesive mucosal therapeutic system containing at least one active principle, with an active principle dissolution test of more than 70% over 8 hours and to a method for its preparation. Said bioadhesive therapeutic system comprises quantities of natural proteins representing at least 50% by weight of active principle and at least 20% by weight of said tablet, between 10% and 20% of an hydrophilic polymer, and compression excipients, and comprising between 4% and 10% of an alkali metal alkylsulphate to reinforce the local availability of active principle and between 0.1 % and 1% of a monohydrate sugar.

The below example illustrates the invention without limiting its scope.

### EXAMPLE:

### Case-study:

A 53 year-old female subject, first presented with clinical symptoms of Cushing's syndrome in August 2006, and she was diagnosed with Cushing's syndrome secondary to ectopic ACTH secretion in March 2007. She received 200mg mifepristone, three times a day (in the morning, at noon, and in the evening) for 2.5 weeks before dose reduction for 1 week to 400 mg (200 mg twice a day).

The administration of mifepristone rapidly improved (after 2 weeks of treatment) general clinical consequences of hypercortisolism: glycemia returned to normal, insulin was stopped and dose of metformin decreased by two. The dose of enalapril previously administered for hypertension was decreased from 30mg to 10 mg.

### References

- Attardi et al, Journal of Steroid Biochemistry & Molecular Biology, 2004, 88: 277-288
- Beaufrere et al, The Lancet, July 25, 1987, page 217
- Chrousos et al, "Clinical applications of RU486, a prototype glucocorticoid and progestin antagonist in : Adrenal and hypertension" Eds F. mantero, BA Scoggins, R. Takeda, EG Biglieri" JW Funder, Raven Press (NY), 1989, pp273-84
- Chu et al, 2001, J Clin Endocrinol Metab, 86:3568-73
- Dahia and Grossman, 1999, Endocr. Rev. 20:136-55
- Doppman et al, 1987, Radiology, 163:501-3
- Doppman et al, 1989, Radiology, 172:115-24
- Meier and Biller, 1997, Endocrinol Metab Clin North Am 26:741-762
- Newell-Price et al, 1998, Endocr Rev, 19:647-72
- Newfield et al, 2000; J Clin Endocrinol Metab, 2000, 85:14-21
- Nieman et al, 1985; J Clin Endocrinol Metab, 1985, 61:536-40
- Orth, 1995, N. Engl. J. Med. 332:791-803
- Pass et al, 1990, Ann Thorac Surg, 50 :52-7
- Raux-Demay et al, 1990; J Clin Endocrinol Metab, 1990,70 :230-33
- van der Lely, 1991, Ann Intern Med, 114:143-144
- Wajchenberg et al, 1994, Endocr Rev, 15:752-87

## Claims

1. A pharmaceutical composition comprising a mifepristone, for use in treating Cushing's syndrome in an adult or an adolescent patient, wherein said pharmaceutical composition is suitable for oral administration, and is administered at least three times a day, wherein the total daily amount of mifepristone administered is inferior or equal to 600mg.

2. The composition for use according to claim 1, wherein the total daily amount of mifepristone administered is less than about 20mg/kg/day.

## Patentansprüche

1. Eine pharmazeutische Zusammensetzung umfassend Mifepriston zur Verwendung bei der Behandlung des Cushing-Syndroms in einem erwachsenen oder adoleszenten Patienten, wobei die pharmazeutische Zusammensetzung für die orale Verabreichung geeignet ist und mindestens dreimal pro Tag verabreicht wird, wobei die verabreichte Tagesgesamtmenge an Mifepriston weniger als oder genau 600 mg beträgt.

2. Die Zusammensetzung zur Verwendung nach Anspruch 1, wobei die Tagesgesamtmenge an Mifepriston weniger als etwa 20mg/kg/Tag beträgt.

## Revendications

1. Composition pharmaceutique comprenant une mifépristone, pour une utilisation dans le traitement du syndrome de Cushing chez un patient adulte ou adolescent, ladite composition pharmaceutique étant appropriée pour une administration orale, et étant administrée au moins trois fois par jour, la quantité journalière totale de mifépristone administrée étant inférieure ou égale à 600 mg.

2. Composition pour une utilisation selon la revendication 1, la quantité journalière totale de mifépristone administrée étant inférieure à environ 20 mg/kg/jour.
